# EUROPÄISCHE PATENTSCHRIFT

(11) **EP 2 557 071 B1**
(45) Veröffentlichungstag und Bekanntmachung des Hinweises auf die Patenterteilung: **04.11.2015**
(21) Anmeldenummer: 11009115.4
(22) Anmeldetag: 17.11.2011
(51) Int. Cl.: C07C 7/00, C07C 7/04, C07C 7/11, C10G 70/04

(54) **Trennsequenz für Kohlenwasserstoffe aus milder thermischer Spaltung**
Separating sequence for hydrocarbons from mild thermal splitting
Séquence de séparation pour hydrocarbures à partir d'une séparation thermique douce

(30) Priorität: 11.08.2011 DE 102011110003
(43) Veröffentlichungstag der Anmeldung: 13.02.2013
(73) Patentinhaber: Linde Aktiengesellschaft, 80331 München (DE)
(72) Erfinder: Duc, Tuat Pham, 82377 Penzberg (DE); Schmidt, Gunther, 82041 Deisenhofen (DE); Schmigalle, Holger, 82515 Wolfratshausen (DE); Walter, Stefanie, Dr., 82418 Seehausen (DE)
(74) Vertreter: Galander, Marcus

(56) Entgegenhaltungen:
- DE-A1-102006 010 519
- DE-A1-102009 038 456

## Beschreibung

Die Erfindung betrifft ein Verfahren zur Trennung von Kohlenwasserstoffen in einer Anlage zur Erzeugung von Kohlenwasserstoffen aus einem flüssigen kohlenwasserstoffhaltigen Einsatz mittels Spaltung,
- wobei das als Rohgas entstehende, gasförmige Kohlenwasserstoffe enthaltende, Produktgas der Spaltung verdichtet und getrocknet wird,
- und als Einsatzstoff in eine Trennstufe (im Folgenden: Front-End C3/C4-Trennung) geführt wird,
- in welcher das Rohgas in eine Kohlenwasserstofffraktion aus Kohlenwasserstoffen mit maximal 3 Kohlenstoffatomen und eine Kohlenwasserstofffraktion aus Kohlenwasserstoffen mit mindestens 4 Kohlenstoffatomen getrennt wird,
- wobei die Front-End C3/C4-Trennung verfahrenstechnisch einen C4-Absorber und einen Depropanizer aufweist,
- wobei eine Kohlenwasserstofffraktion aus Kohlenwasserstoffen mit maximal 3 Kohlenstoffatomen als gasförmiges Kopfprodukt des C4-Absorbers gewonnen wird,
- und wobei eine flüssige Kohlenwasserstofffraktion aus Kohlenwasserstoffen mit mindestens 4 Kohlenstoffatomen als Sumpfprodukt des Depropanizers gewonnen wird.

In einer Anlage zur Erzeugung von Kohlenwasserstoffen, einer sogenannten Olefinanlage, werden die Kohlenwasserstoffe oder Olefine durch Spaltung kohlenwasserstoffhaltiger Einsätze erzeugt. Die kohlenwasserstoffhaltigen Einsätze liegen dabei entweder in der flüssigen oder gasförmigen Phase vor und werden mittels thermischer oder katalytischer Spaltung mit oder ohne Dampf in kürzerkettige Kohlenwasserstoffe umgewandelt. Das bei der Spaltung entstehende Gemisch aus vorwiegend kürzerkettigen Olefinen wird als Spaltgas oder Rohgas bezeichnet. Bei der Spaltung eines flüssigen kohlenwasserstoffhaltigen Einsatzes wird das Rohgas zumeist als Einsatz in eine Ölwäsche geführt. In der Ölwäsche wird das Rohgas abgekühlt und verbliebene längerkettige Kohlenwasserstoffe, wie beispielsweise Kokspartikel und schwere Ölkomponenten, aus dem Rohgas heraus gewaschen. Anschließend wird das Rohgas zur weiteren Reinigung und Abkühlung in eine Wasserwäsche geführt und in der Rohgasverdichtung verdichtet. Bei der Spaltung eines gasförmigen kohlenwasserstoffhaltigen Einsatzes kann zumeist auf die Ölwäsche verzichtet werden. Anschließend wird das Rohgas nach dem Stand der Technik in einer Laugewäsche von weiteren Verunreinigungen, wie Kohlendioxid und Schwefelwasserstoff, befreit und getrocknet.

Das gereinigte und getrocknete Rohgas besteht nunmehr aus einem Gemisch der gewünschten Olefinprodukte und Beiprodukte. Um die gewünschten Olefinprodukte verwerten zu können, muss das Gemisch in die einzelnen Olefinbestandteile getrennt werden.

Eine derartiges Verfahren zur Trennung von Kohlenwasserstoffen beginnt nach dem Stand der Technik entweder mit einer Trennstufe, in der Olefine mit höchstens 2 Kohlenstoffatomen von Olefinen mit mindestens 3 Kohlenstoffatomen getrennt werden (Front-End C₂/ C₃-Trennung), oder einer Trennstufe, in der Olefinen mit höchstens 3 Kohlenstoffatomen von Olefinen mit mindestens 4 Kohlenstoffatomen (Front-End C₃/ C₄-Trennung) getrennt werden.

Beginnt die Trennsequenz mit einer Front-End C₂/ C₃-Trennung wird die entstehende Olefinfraktion mit höchstens 2 Kohlenstoffatomen (C₂ -Fraktion) nach einer katalytischen Hydrierung zur Entfernung von Azetylen zu einem Tieftemperaturzerlegungsteil geleitet, wo sie in ihren einzelnen Fraktionen zerlegt wird. Die C₂ -Fraktion wird dabei von der Methan- und Wasserstofffraktion getrennt. Die verbleibende Fraktion aus Kohlenwasserstoffen mit mindestens 3 Kohlenstoffatomen (C₃₊-Fraktion) wird in eine Trennstufe geführt (Depropanizer), in der als Sumpfprodukt eine Fraktion aus Kohlenwasserstoffen mit mindestens 4 Kohlenstoffatomen (C₄₊-Fraktion) gewonnen wird. Über Kopf wird im Depropanizer eine Olefinfraktion aus Kohlenwasserstoffen mit 3 Kohlenstoffatomen (C₃-Fraktion) gewonnen. Die C₃-Fraktion wird anschließend ebenfalls vor ihrer Weiterverarbeitung katalytisch hydriert.

Im Rahmen dieser Anmeldung wird eine Kohlenwasserstofffraktion, die aus Kohlenwasserstoffen besteht, die n Kohlenstoffatome aufweisen, als Cₙ-Fraktion bezeichnet. Besteht diese Kohlenwasserstofffraktion aus Kohlenwasserstoffen, die mindestens n Kohlenstoffatome aufweisen, wird die Kohlenwasserstofffraktion als Cₙ₊-Fraktion bezeichnet. Eine Fraktion aus Kohlenwasserstoffen mit maximal n Kohlenstoffatomen wird als Cₙ₋-Fraktion bezeichnet. Dabei steht n für die natürlichen Zahlen 1, 2, 3, 4...

Eine Trennstufe, bei der Kohlenwasserstoffe mit 2 oder mehr Kohlenstoffatomen als flüssiges Sumpfprodukt gewonnen wird, wird im Rahmen dieser Anmeldung als Demethanizer bezeichnet. Eine Trennstufe mit einer C₃₊-Fraktion als Sumpfprodukt wird als Deethanizer bezeichnet. Entsprechend wird eine Trennstufe mit einer C₄₊-Sumpffraktion als Depropanizer bezeichnet.

Bei einer Trennsequenz nach dem Stand der Technik, die mit einer Front-End C₃/ C₄-Trennung beginnt, erhält man bei dem Druck des verdichteten Rohgases eine C₃₋-Fraktion und eine C₃₊-Fraktion. Bei dem herrschenden vollen Rohgasdruck ist eine scharfe Trennung in eine C₃₋-Fraktion und eine C₄₊-Fraktion nach dem Stand der Technik nicht möglich, da die Sumpftemperatur so hoch wäre, dass verstärkt Polymerbildung und somit eine ungewünschte Belagsbildung auftreten würde. In der weiteren Trennsequenz nach dem Stand der Technik wird die C₃₋-Fraktion nach einer katalytischen Hydrierung zu einer C₂/ C₃-Trennung geführt. Die C₃₋-Fraktion wird in eine C₃-Fraktion und eine C₂₋-Fraktion getrennt. Die C₄₊-Fraktion wird zu einer C3/C4-Trennung geleitet, wo sie in eine C₃-Fraktion sowie eine C₄₊-Fraktion getrennt und die entstandene C₃-Fraktion muss anschließend katalytisch hydriert werden.

Somit sind nach dem Stand der Technik sowohl bei einer Trennsequenz mit einer Front-End C₂/ C₃-Trennung als auch bei einer Trennsequenz mit einer Front-End C₃/ C₄-Trennung 2 unabhängige katalytische Hydrierungsstufen mit dem entsprechenden Rohr-und Festbettreaktor notwendig.

In DE 102006010519 wird ein alternatives Verfahren zur Trennung von Olefinen vorgeschlagen. DE 102006010519 offenbart eine Trennsequenz mit einem bei vollem Rohgasdruck arbeitenden C₄-Absorber und einen Depropanizer, der bei einem Druck von 8 - 12 bar betrieben wird. Durch die Kombination aus C₄-Absorber und Depropanizer werden die Olefine in eine C₃₋-Fraktion und eine C₄₊-Fraktion getrennt. Die C₃₋-Fraktion wird dann vollständig verdichtet und zur katalytischen Hydrierung geführt, während die C₄₊-Fraktion zur Weiterverarbeitung ausgeführt wird. Die C₃₋-Fraktion wird nach der katalytischen Hydrierung einer C₂/ C₃-Trennung unterzogen und in eine C₂₋-Fraktion und eine C₃-Fraktion getrennt. Die C₂₋-Fraktion wird als Einsatz in den Tieftemperaturtrennungsteil weitergeleitet, während die C₃-Fraktion zur Weiterverarbeitung geführt wird.

Eine ähnliche Trennsequenz für einen flüssigen kohlenwasserstoffhaltigen Einsatz wird in der DE 102009038456 beschrieben. Bei der hier offenbarten Trennsequenz wird ebenfalls ein C₄-Absorber mit einem Depropanizer kombiniert, wobei der C₄-Absorber bei vollem Rohgasdruck und der Depropanizer bei einem Druck zwischen 8 bar und 12 bar betrieben wird. DieC₃₋-Fraktion wird nach der katalytischen Hydrierung einer C₂/ C₃-Trennung unterzogen, wobei die C₃₋-Fraktion in einer ersten Kolonne in eine C₂₋-Fraktion und eine C₂/C₃-Fraktion getrennt wird. Die weitere Trennung erfolgt in einer zweiten Kolonne mit zwei verfahrenstechnisch getrennten Abschnitten, wobei der obere Abschnitt als Demethanizer und der untere Abschnitt als Deethanizer ausgeführt ist. Im Demethanizer werden gelöstes Methan und gelöster Wasserstoff aus den Kondensaten der Tiefkühlung ausgestrippt. Im Deethanizer entsteht eine C₂₋-Fraktion und eine C₃-Fraktion. Die C₃-Fraktion C3 wird dabei als Sumpfprodukt gewonnen. Als Rücklauf dient ein Teil der flüssigen C₂-Fraktionaus dem Demethanizer. Der Sumpf des Deethanizers wird entweder mit Rohgas oder mit warmem C₃-Kältemittel oder mit warmem Gemischkältemittel aufgekocht. Die C₂-Fraktionen aus der zweiten Kolonne, welche als Gas- und flüssiges Produkt seitlich aus dem Sumpf des Demethanizers der zweiten Kolonne abgezogen werden, werden einem C₂-Splitter aufgegeben. Dort wird Ethylen als Kopfprodukt gewonnen.

Der vorliegenden Erfindung liegt die Aufgabe zugrunde, ein Verfahren der eingangs genannten Art derart auszugestalten, dass der energetische und apparative Aufwand zur Trennung von Kohlenwasserstoffen minimiert wird. Dabei soll insbesondere der energetische Aufwand bei der Trennung eines bei der Spaltung entstehenden Rohgases minimiert werden, welches ein Verhältnis von Ethylen zu Propylen von nahezu 1, insbesondere unter 1 aufweist.

Die gestellte Aufgabe wird durch die Merkmalskombination des unabhängigen Anspruches 1 gelöst. Vorteilhafte Ausgestaltungen der Erfindung werden in den Unteransprüchen angegeben.

Erfindungsgemäß beginnt die Trennsequenz der Kohlenwasserstoffprodukte des Rohgases mit einer Front-End C3/ C₄-Trennung, welche eine weitere verfahrenstechnische C2/C4-Trennstufe umfasst. Diese weitere C2/ C4-Trennstufe wird zwischen dem C4-Absorber und dem Depropanizer angeordnet.

Bei einem höheren Anteil von längerkettigen Kohlenwasserstoffen im Rohgas, d.h. konkret bei einem Rohgas mit hohem C₄₊ -Anteil, steigt während der Verdichtung des Rohgases sowohl die Kondensatmenge als auch der Anteil der gelösten C₂₋-Komponenten in den Kondensaten an, so dass eine scharfe Front-End C3/C4-Trennung schwieriger und energetisch aufwendiger wird. Durch die vorliegende Erfindung wird dieses Problem jedoch komplett gelöst.Durch die zusätzliche C2/C4-Trennstufe wird der Kältebedarf vor allem des Kopfproduktes des Depropanizers verringert und somit der Energieaufwand der Trennsequenz minimiert.

Erfindungsgemäß wird zwischen dem C4-Absorber und dem Depropanizer eine weitere C2/C4-Trennstufe angeordnet. In dieser Trennstufe findet eine Trennung von C₂₋-Komponenten und C₄₊-Komponenten statt, wobei sich die C₃-Komponenten in dieser Trennstufe sowohl im gasförmigen Kopf als auch im flüssigen Sumpfprodukt finden, so dass das Kopfgas dieser Trennstufe bei einem höheren Temperaturniveau kondensiert werden kann. Dadurch wird eine Temperaturerhöhung in einem Bereich der verstärkten Polymerbildung im Sumpfprodukt vermieden.

Die gesamte erfindungsgemäße Front-End C3/C4-Trennung bestehend aus dem C4-Absorber, der C2/C4-Trennstufe und dem Depropanizer, erlaubt insgesamt eine scharfe verfahrenstechnische Trennung des bei der Spaltung entstandenen und verdichteten Rohgases in eine C₃₋-Fraktion und in eine C₄₊ -Fraktion. Dabei werden die Temperaturen durchgängig in Bereichen gehalten, bei denen keine Polymer- oder Belagsbildung auftritt. Durch die erfindungsgemäße Kombination des C4-Absorbers, der C2/C4-Trennstufe und dem Depropanizer sowie durch unscharfe Trennung kann sukzessive der Druck der anfallenden Kondensate während des Trennprozesses so weit reduziert werden, dass im Depropanizer eine C₄₊ Fraktion anfällt, die keine Kohlenwasserstoffe mit weniger als vier Kohlenstoffatomen enthält, ohne dass dabei die Sumpftemperatur derart steigt, dass Polymer- oder Belagsbildung auftritt, und wobei der energetische Aufwand für die Trennung minimiert wird.

In einer bevorzugten Ausgestaltung der Erfindung werden der C4-Absorber und die C2/C4-Trennstufe bei einem Druck zwischen 18 und 20 bar betrieben, wobei bevorzugt die C2/C4-Trennstufe bei etwas höhrerem Druck als der C4-Absorber betrieben wird. Diese Ausgestaltung der Erfindung erlaubt das verdichtete Rohgas bei vollem Rohgasdruck direkt dem C4-Absorber aufzugeben.

In einer bevorzugten Ausgestaltung der Erfindung werden der C4-Absorber und die C2/C4-Trennstufe in einer Kolonne vereinigt. In dieser Ausgestaltung werden C4-Absorber und C2/C4-Trennstufe in einer Kolonne zusammengefasst, bilden aber verfahrenstechnisch getrennte Abschnitte dieser Kolonne. Entsprechend ist das Druckniveau beider verfahrenstechnischer Abschnitte (C4-Absorber, C2/C4-Trennstufe) in dieser Ausgestaltung der Erfindung gleich.

In einer alternativen Ausgestaltung der Erfindung sind C4-Absorber und C2/C4-Trennstufe verschiedene Kolonnen. In dieser alternativen Ausgestaltung der Erfindung werden die verfahrenstechnisch klar getrennten C4-Absorber und die C2/C4-Trennstufe auch apparativ in zwei separate Kolonnen aufgeteilt. In dieser Ausgestaltung der Erfindung ist eine Sumpfpumpe für den C4-Absorber erforderlich. Die C2/C4-Trennstufe wird bei etwas höherem Druck als der C4-Absorber betrieben.

Vorteilhafterweise wird das Sumpfprodukt der C2/C4-Trennstufe dem Depropanizer aufgegeben. Die C2/C4-Trennstufe trennt die Kohlenwasserstoffe in eine C₃₋-Fraktion und eine C₃₊-Fraktion. Die C₃₊-Fraktion fällt dabei als Sumpfprodukt an. Dieses Sumpfprodukt wird vorteilhafterweise dem Depropanizer aufgegeben, wo die C₃-Fraktion abgetrennt wird und eine reine C₄₊-Fraktion als Sumpfprodukt gewonnen wird.

Der Depropanizer wird bevorzugt bei einem Druck zwischen 10 und 12 bar betrieben.

Besonders bevorzugt enthält das zu trennende Rohgas Ethylen und Propylen in einem Verhältnis von 1 oder kleiner 1. Dabei wurden die Bedingungen der Spaltung entsprechend eingestellt. Die Vorteile der Erfindung kommen besonders zum Tragen, wenn die Bedingungen der Spaltung derart eingestellt werden, dass der Propylenanteil im Rohgas dem Ethylenanteil entspricht oder größer als der Ethylenanteil ist. Bei einem derartigen Rohgas gelingt es durch die Erfindung insbesondere, eine Front-End-C₃/C₄Trennung scharf durchzuführen, ohne dass es in den beteiligten Apparaten zu Polymer- oder Belagbildung kommt.

Bevorzugt wird ein Verhältnis von Ethylen zu Propylen von 1 oder kleiner 1 dadurch erreicht, dass dem im wesentlichen aus gesättigten Kohlenwasserstoffen bestehenden flüssigen kohlenwasserstoffhaltigen Einsatz vor der thermischen Spaltung ein gewisser Anteil gesättigter Kohlenwasserstoffe (bevorzugt zwischen 3 Gew-% und 40 Gew-%, besonders bevorzugt zwischen 5 Gew-% und 30 Gew-%, insbesondere zwischen 15 Gew-% und 25 Gew-%) zugemischt wird. Die thermische Spaltung findet dabei zweckmäßigerweise bei milden Bedingung (bevorzugt 740°C - 800°C und/oder 2.5 bar abs - 4 bar abs) statt. Vorteilhafterweise werden dem flüssigen Einsatz dabei ein oder mehrere rückgeführte C₄₊-Fraktion/en als gesättigte Kohlenwasserstoffe zugemischt. Die vorliegende Erfindung ist besonders zur Trennung von Kohlenwasserstoffen in einer Anlage zur Erzeugung von Kohlenwasserstoffen aus einem flüssigen kohlenwasserstoffhaltigen Einsatz mittels Spaltung geeignet.

Mit der vorliegenden Erfindung gelingt es insbesondere, den energetischen Aufwand einer Trennsequenz für ein Rohgas, wie es insbesondere bei der Spaltung von flüssigen kohlenwasserstoffhaltigen Einsätzen entsteht, zu minimieren. Speziell bei einem Rohgas mit einem höheren C₄₊-Anteil kann bei der erfindungsgemäßen Front-End-C3/C4-Trennung eine scharfe Trennung in eine C₃₋-Fraktion und eine C₄₊-Fraktion des Rohgases erreicht werden, ohne dass es in den beteiligten Apparaten zu einer erhöhten Polymer- oder Belagbildung kommt.

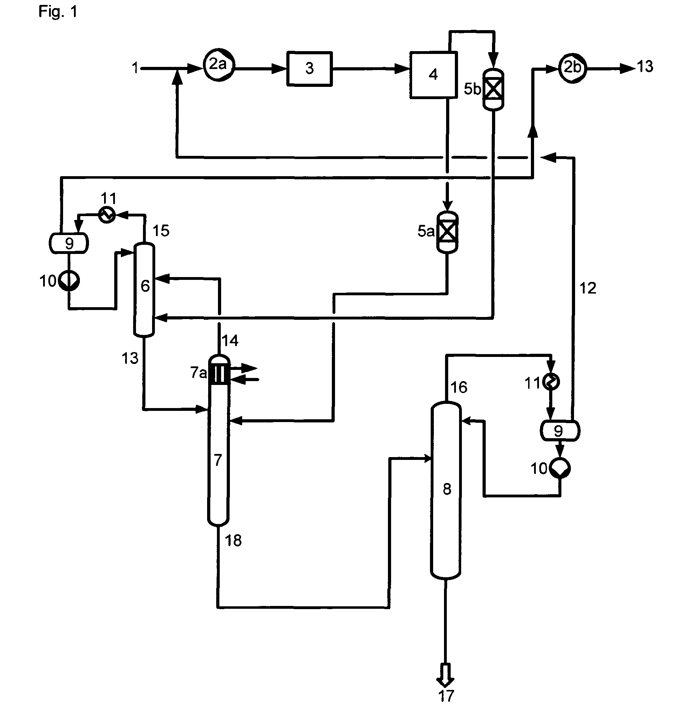

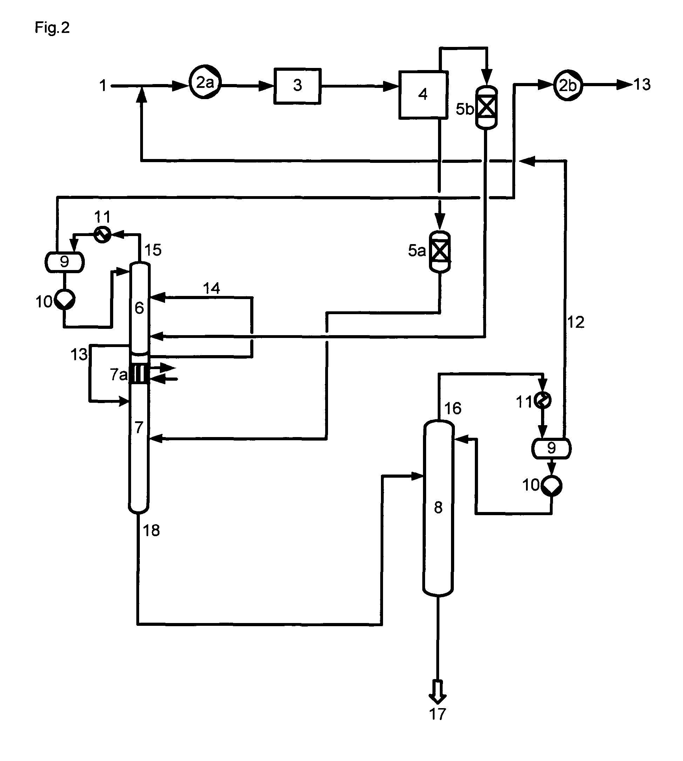

Im Folgenden soll die Erfindung anhand der beiden in den Figuren 1 und 2 dargestellten Ausführungsbeispielen näher erläutert werden.

Es zeigen:
- Figur 1: Eine Ausgestaltung der Erfindung, bei der C4-Absorber und C2/C4-Trennstufe verschiedene Kolonnen sind.
- Figur 2: Eine alternative Ausgestaltung der Erfindung, bei der C4-Absorber und C2/C4-Trennstufe in einer Kolonne angeordnet sind.

Figur 1 zeigt eine Ausgestaltung des erfindungsgemäßen Verfahrens zur Trennung von Kohlenwasserstoffen in einer Anlage, in der ein flüssiger kohlenwasserstoffhaltiger Einsatz derart gespalten wird, dass das bei der Spaltung entstehende Rohgas einen Ethylen zu Propylenanteil von maximal 1 oder kleiner aufweist. Das als Spaltprodukt entstehende Rohgas 1 wird als Einsatz in eine Ölwäsche (nicht dargestellt) geführt. In der Ölwäsche wird das Rohgas abgekühlt und verbliebene längerkettige Kohlenwasserstoffe, wie beispielsweise Kokspartikel und schwere Ölkomponenten, aus dem Rohgas herausgetrennt. Anschließend wird das Rohgas 1 zur weiteren Reinigung und Abkühlung in eine Wasserwäsche (nicht dargestellt) geführt und von dort in eine drei-stufige Rohgasverdichtung 2a geführt. In der dreistufigen Rohgasverdichtung 2a wird das Rohgas auf einen Druck von ca. 20 bar, bevorzugt 19 bar, verdichtet und in einer Wäsche 3 von Sauergaskomponenten, wie Kohlendioxid und Schwefelwasserstoff, befreit. Anschließend wird das gereinigte Rohgas in einer Vorkühlung 4 vorgekühlt und über die beiden Trockner 5a und 5b getrocknet. Anschließend beginnt die eigentliche Trennsequenz des Rohgases 1.

Durch die Rohgasverdichtung 2a wird das Rohgas erwärmt, so dass eine Abkühlung vor der Sauergaswäsche 3 notwendig wird (nicht dargestellt). Das dabei entstehende Kondensat wird direkt in den Depropanizer 8 geführt (nicht dargestellt). Die dabei dem Depropanizer 8 zwangsläufig zugeführten Sauergaskomponenten verlassen jedoch den Depropanizer 8 mit der Gasphase 16 über Kopf und werden zur Rohgasverdichtung 2a zurückgeführt 12. Die in diesem Abschnitt geschilderten Kondensate können dabei von jeder Stufe der Rohgasverdichtung 2a abgezogen werden, bevorzugt jedoch wie in diesem Ausführungsbeispiel nach der 3. Stufe der Rohgasverdichtung 2a.

Das in der Vorkühlung 4 entstehende Kondensat wird über den Trockner 5a der C2/C4-Trennstufe 7 aufgegeben. Das in der Vorkühlung 4 entstehende Kopfgas wird über den Trockner 5b direkt dem C4-Absorber 6 aufgegeben. Sowohl C4-Absorber 6 als auch die C2/C4-Trennstufe 7 arbeiten dabei bei dem vollen Rohgasdruck zwischen 18 und 20 bar, bevorzugt 19 bar. Der Druck in dem C4-Absorber 6 ist leicht geringer als der Druck in der C2/C4-Trennstufe 7. In dem C4-Absorber 6 entsteht als gasförmiges Kopfprodukt eine reine C₃₋-Fraktion 15. Diese wird in einer vierten Verdichtungsstufe 2b weiter verdichtet und zur weiteren Zerlegung 13 geführt, bei der dann aus dieser C₃₋-Fraktion15 die Wertprodukte Ethylen und Propylen herausgetrennt werden.

Das Sumpfprodukt 13 des C4-Absorbers 6 wird ebenso wie das Kondensat vom Trockner 5a der C2/C4-Trennstufe 7 aufgegeben. In der C2/C4-Trennstufe 7 werden die Kohlenwasserstoffe mit drei Kohlenstoffatomen auf das gasförmige Kopfprodukt und das flüssige Sumpfprodukt verteilt. Dabei enthält das Kopfprodukt nur wenig Kohlenwasserstoffen mit mehr als drei Atomen und das Sumpfprodukt ist frei von Kohlenwasserstoffen mit weniger als zwei Kohlenstoffatomen. Somit wird in der C2/C4-Trennstufe 7 ein gasförmiges Kopfprodukt gewonnen, welches eine C₄₋-Fraktion 14 darstellt. Das flüssige Sumpfprodukt 18 der C2/C4-Trennstufe 7 ist eine C₃₊-Fraktion 18. Um sicherzustellen, dass das Kopfprodukt 14 der C2/C4-Trennstufe 7 nur wenige Kohlenwasserstoffen mit vier oder mehr Kohlenstoffatomen enthält, weist die C2/C4-Trennstufe 7 einen Kopfkondensator 7a auf.

Das Sumpfprodukt 18 der C2/C4-Trennstufe 7 wird zur Abtrennung der C₄₊-Fraktion dem Depropanizer 8 aufgegeben. Der Depropanizer 8 arbeitet bei einem Druck zwischen 8 und 12 bar. Das Sumpfprodukt 18 der C2/C4-Trennstufe 7 wird daher in den Depropanizer 8 entspannt. Im Depropanizer 8 bildet sich eine reine C₄₊-Fraktion als Sumpfprodukt 17 und wird von diesem abgezogen. Das Sumpfprodukt 17 des Depropanizers ist dabei frei von jeglichen C₃₋-Komponenten. Als Kopfprodukt 16 des Depropanizers wird eine Fraktion gewonnen, die hauptsächlich aus Kohlenwasserstoffen mit drei Kohlenstoffatomen besteht. Über einen Wärmetauscher 11 wird das Kopfprodukt 16 des Depropanizers 8 weiter abgekühlt und in einen Abscheider 9 geführt. Die im Abscheider gewonnene Gasphase 12 wird in das Rohgas 1 vor die dreistufige Verdichtung 2a zurückgeführt. Das flüssige Produkt des Abscheiders 9 wird über eine Pumpe 10 als Rücklauf dem Depropanizer 8 aufgegeben.

Ebenso wird das gasförmige Kopfprodukt 15 des C4-Absorbers 6 über einen Wärmetauscher 11 abgekühlt und in einen Abscheider 9 geführt. Die dabei entstehende flüssige Phase wird über die Pumpe 10 dem C4-Absorber 6 wieder als Rücklauf aufgegeben. Die gasförmige Phase wird in der vierten Stufe der Rohgasverdichtung 2b verdichtet und als C₃₋-Fraktion zur Ethylen- und Propylengewinnung 13 geführt.

Figur 2 funktioniert ähnlich wie das Ausführungsbeispiel gemäß Figur 1. Gleiche Teile wurden wie in Figur 1 mit den gleichen Bezugszeichen gekennzeichnet. Im Unterschied zu dem Ausführungsbeispiel gemäß Figur 1 ist jedoch hier C4-Absorber 6 und C2/C4-Trennstufe 7 in einer Kolonne angeordnet.

## Patentansprüche

1. Verfahren zur Trennung von Kohlenwasserstoffen in einer Anlage zur Erzeugung von Kohlenwasserstoffen aus einem kohlenwasserstoffhaltigen Einsatz mittels Spaltung,
• wobei das als Rohgas (1) entstehende, gasförmige Kohlenwasserstoffe enthaltende, Produktgas der Spaltung verdichtet (2a) und getrocknet (5a,5b) wird,
• und als Einsatzstoff in eine Trennstufe (im Folgenden: Front-End C3/C4-Trennung) geführt wird,
• in welcher das Rohgas (1) in eine Kohlenwasserstofffraktion aus Kohlenwasserstoffen mit maximal 3 Kohlenstoffatomen (15) und eine Kohlenwasserstofffraktion aus Kohlenwasserstoffen mit mindestens 4 Kohlenstoffatomen (17) getrennt wird,
• wobei die Front-End C3/C4-Trennung verfahrenstechnisch einen C4-Absorber (6) und einen Depropanizer (8) aufweist,
• wobei eine Kohlenwasserstofffraktion aus Kohlenwasserstoffen mit maximal 3 Kohlenstoffatomen als gasförmiges Kopfprodukt (15) des C4-Absorbers (6) gewonnen wird,
• und wobei eine flüssige Kohlenwasserstofffraktion aus Kohlenwasserstoffen mit mindestens 4 Kohlenstoffatomen als Sumpfprodukt (17) des Depropanizers (8) gewonnen wird, **dadurch gekennzeichnet, dass**
• die Front-End C3/C4-Trennung einen weitere verfahrenstechnische C2/C4-Trennstufe (7) umfasst,
• wobei die C2/C4-Trennstufe (7) zwischen C4-Absorber (6) und Depropanizer (8) angeordnet ist.

2. Verfahren nach Anspruch 1, **dadurch gekennzeichnet, dass** der C4-Absorber (6) und die C2/C4-Trennkolonne (7) bei einem Druck zwischen 18 bar und 20 bar betrieben werden, wobei bevorzugt die C2/C4-Trennstufe (7) bei etwas höherem Druck als der C4-Absorber (6) betrieben wird.

3. Verfahren nach Anspruch 1 oder 2, **dadurch gekennzeichnet, dass** der C4-Absorber (6) und die C2/C4-Trennstufe (7) in einer Kolonne vereinigt sind.

4. Verfahren nach einem der Ansprüche 1 oder 2, **dadurch gekennzeichnet, dass** der C4-Absorber (6) und die C2/C4-Trennstufe (7) verschiedene Kolonnen sind.

5. Verfahren nach einem der Ansprüche 1 bis 4, **dadurch gekennzeichnet, dass** das Sumpfprodukt (18) der C2/C4-Trennstufe (7) dem Depropanizer (8) aufgegeben wird.

6. Verfahren nach einem der Ansprüche 1 bis 5, **dadurch gekennzeichnet, dass** der Depropanizer (8) bei einem Druck zwischen 10 bar und 12 bar betrieben wird.

7. Verfahren nach einem der Ansprüche 1 bis 6, **dadurch gekennzeichnet, dass** als Sumpfprodukt (17) des Depropanizers (8) eine Kohlenwasserstofffraktion mit mindestens 4 Kohlenstoffatomen gewonnen wird.

8. Verfahren nach einem der Ansprüche 1 bis 7, **dadurch gekennzeichnet, dass** das Rohgas (1) Ethylen und Propylen in einem Verhältnis von 1 oder kleiner 1 enthält, wobei die Bedingungen der Spaltung entsprechend eingestellt wurden.

## Claims

1. Process for separating hydrocarbons in a plant for producing hydrocarbons from a hydrocarbonaceous input by means of cracking,
• wherein the product gas from the cracking which comprises gaseous hydrocarbons and is formed as cracked gas (1) is compressed (2a) and dried (5a, 5b),
• and is conducted as a feedstock into a separation stage (hereinafter: front end C3/C4 separation),
• in which the cracked gas (1) is separated into a hydrocarbon fraction composed of hydrocarbons having not more than 3 carbon atoms (15) and a hydrocarbon fraction composed of hydrocarbons having at least 4 carbon atoms (17),
• wherein the front end C3/C4 separation, in terms of process technology, has a C4 absorber (6) and a depropanizer (8),
• wherein a hydrocarbon fraction composed of hydrocarbons having not more than 3 carbon atoms is obtained as a gaseous top product (15) from the C4 absorber (6),
• and wherein a liquid hydrocarbon fraction composed of hydrocarbons having at least 4 carbon atoms is obtained as a bottom product (17) of the depropanizer (8), **characterized in that**
• the front end C3/C4 separation comprises a further process stage of a C2/C4 separation stage (7),
• wherein the C2/C4 separation stage (7) is arranged between C4 absorber (6) and depropanizer (8).

2. Process according to Claim 1, **characterized in that** the C4 absorber (6) and the C2/C4 separating column (7) are operated at a pressure between 18 bar and 20 bar, the C2/C4 separation stage (7) preferably being operated at somewhat higher pressure than the C4 absorber (6).

3. Process according to Claim 1 or 2, **characterized in that** the C4 absorber (6) and the C2/C4 separation stage (7) are combined in one column.

4. Process according to either of Claims 1 and 2, **characterized in that** the C4 absorber (6) and the C2/C4 separation stage (7) are different columns.

5. Process according to any of Claims 1 to 4, **characterized in that** the bottom product (18) of the C2/C4 separation stage (7) is supplied to the depropanizer (8).

6. Process according to any of Claims 1 to 5, **characterized in that** the depropanizer (8) is operated at a pressure between 10 bar and 12 bar.

7. Process according to any of Claims 1 to 6, **characterized in that** the bottom product (17) obtained from the depropanizer (8) is a hydrocarbon fraction having at least 4 carbon atoms.

8. Process according to any of Claims 1 to 7, **characterized in that** the cracked gas (1) contains ethylene and propylene in a ratio of 1 or less than 1, the cracking conditions having been set correspondingly.

## Revendications

1. Procédé pour la séparation d'hydrocarbures dans une installation pour produire des hydrocarbures, à partir d'une charge contenant des hydrocarbures, par dissociation,
- le gaz produit de la dissociation, formé comme gaz brut (1), contenant des hydrocarbures gazeux, étant compressé (2a) et séché (5a,5b),
- et introduit comme substance de départ dans un étage de séparation (dans la suite : séparation C3/C4 Front-End),
- dans lequel le gaz brut (1) est séparé en une fraction hydrocarbonée constituée d'hydrocarbures comprenant au maximum 3 atomes de carbone (15) et en une fraction hydrocarbonée constituée d'hydrocarbures comprenant au moins 4 atomes de carbone (17),
- la séparation C3/C4 Front-End présentant, d'un point de vue technique de procédé, un absorbant de C4 (6) et un appareil de dépropanisation (8),
- une fraction hydrocarbonée constituée d'hydrocarbures comprenant au maximum 3 atomes de carbone étant obtenue comme produit de tête gazeux (15) de l'absorbant de C4 (6),
- et une fraction hydrocarbonée liquide constituée d'hydrocarbures comprenant au moins 4 atomes de carbone étant obtenue comme produit de fond (17) de l'appareil de dépropanisation (8), **caractérisé en ce que**
- la séparation C3/C4 Front-End comprend un autre étage de séparation C2/C4 (7) technique de procédé,
- l'étage de séparation C2/C4 (7) étant disposé entre l'absorbant de C4 (6) et l'appareil de dépropanisation (8).

2. Procédé selon la revendication 1, **caractérisé en ce que** l'absorbant de C4 (6) et la colonne de séparation C2/C4 (7) sont exploités à une pression entre 18 bars et 20 bars, l'étage de séparation C2/C4 (7) étant de préférence exploité à une pression légèrement supérieure à celle de l'absorbant de C4 (6).

3. Procédé selon la revendication 1 ou 2, **caractérisé en ce que** l'absorbant de C4 (6) et l'étage de séparation C2/C4 (7) sont rassemblés dans une colonne.

4. Procédé selon l'une quelconque des revendications 1 ou 2, **caractérisé en ce que** l'absorbant de C4 (6) et l'étage de séparation C2/C4 (7) sont des colonnes différentes.

5. Procédé selon l'une quelconque des revendications 1 à 4, **caractérisé en ce que** le produit de fond (18) de l'étage de séparation C2/C4 (7) est introduit sur l'appareil de dépropanisation (8).

6. Procédé selon l'une quelconque des revendications 1 à 5, **caractérisé en ce que** l'appareil de dépropanisation (8) est exploité à une pression entre 10 bars et 12 bars.

7. Procédé selon l'une quelconque des revendications 1 à 6, **caractérisé en ce qu'**on obtient, comme produit de fond (17) de l'appareil de dépropanisation (8) une fraction hydrocarbonée comprenant au moins 4 atomes de carbone.

8. Procédé selon l'une quelconque des revendications 1 à 7, **caractérisé en ce que** le gaz brut (1) contient de l'éthylène et du propylène dans un rapport de 1 ou inférieur à 1, les conditions de la dissociation ayant été réglées de manière correspondante.
